# EUROPEAN PATENT APPLICATION

(11) **EP 1 930 424 A1**
(43) Date of publication of application: **11.06.2008**
(21) Application number: 06796325.6
(22) Date of filing: 10.08.2006
(51) Int. Cl.: C12N 15/00, A01K 67/027, C07K 14/00, C07K 14/52, C07K 19/00, C12N 5/10

(54) **NOVEL APOPTOSIS INDUCING FACTOR AND METHOD OF INDUCING APOPTOSIS USING THE SAME**

(30) Priority: 22.08.2005 JP 2005240239
(71) Applicant: Kazusa DNA Research Institute Foundation, Kisarazu-shi, Chiba 292-0812 (JP)
(72) Inventor: NAKAYAMA, Manabu, Kisarazu-shi, Chiba 2920812 (JP)
(74) Representative: Webber, Philip Michael
(86) International application number: PCT/JP2006/315826
(87) International publication number: WO 2007/023686

(57) **Abstract**

The purpose of the present invention is to provide a system in which an apoptosis-inducing factor is expressed in an expression promoter-dependent manner, which is not a system wherein a toxin is introduced externally, and a novel protein that can enhance an apoptosis signal used in the system.

The present invention relates to An apoptosis-inducing factor comprising a fusion protein of a polypeptide comprising FADD or a constituting domain thereof and a protein with a self-assembling activity, an expression vector comprising DNA encoding the apoptosis-inducing factor, a transgenic animal having the expression vector introduced therein, an animal wherein apoptosis is induced in a specific cell or tissue and/or at a specific time and a method for producing the animal.

## Description

### FIELD OF THE INVENTION

The present invention is related to a novel apoptosis-inducing factor, a method of inducing apoptosis in a specific cell or tissue and/or at a specific time using the same factor, and experimental animals in which apoptosis is induced in that way.

### BACKGROUND OF THE INVENTION

The presence of model animals for diseases is of a great importance for screening drugs required to treat diseases and confirming effectiveness of the drugs. Once a superior model animal for disease is obtained, a possibility of succeeding in screening of a drug for treatment or alleviation would be significantly increased. It could be even said that it would be just a matter of time before a drug having a certain function would be developed using such model animal. For example, a model animal for diabetes made innumerable contributions to the treatment of diabetes. On the contrary, in the case where there exists no model animal for disease, there is presently no other way than that the effectiveness of a drug will have to be finally confirmed by an actual administration into a patient after its biochemical and cell biological effects are confirmed. The patient would have to take innumerable risk in that case. In order to reduce such risk, the superior model animal for disease is inevitably required.

Also in view of fundamental biology, the presence of a model animal for disease is essential for studying systems that regulate or function not only at a single cell level, but through the whole of an individual animal, such as a cerebral nerve system and allergy-immunity response. Furthermore, in order to study the function of a certain protein or cell type in the whole of the individual animal, it will be necessary to produce not only a model animal for disease that lacks a specific gene, but also a model animal for disease that lacks a specific cell-type so as to study effects exercised to the whole of the individual animal.

Gene targeting method (knockout mouse) has been already developed as a method for studying the function of a certain protein (gene) at an individual level of a mouse. The method deletes a gene encoding a certain protein by means of gene engineering so that the resulting gene-modified mouse (knockout mouse) will not able to produce the same protein. As a result, the function of said protein at an individual level can be revealed by observing a phenotype (pathologic conditions) of the knockout mouse. And, when the mouse unfortunately shows the phenotype, it can be used as a model animal for disease. However, it has been understood now that there is a back-up system that will work at the time of lacking of a gene having an essential function in a body. Actually, there are multiple family genes having identity in an amino acid sequence, or there are systems that do not have any identity regarding an amino acid sequence, but functionally compensate with each other. As a result, a mouse would very often grow normally at first glance even a gene was destroyed. From these reasons, it has been now expected that it would be difficult to obtain a knockout mouse with a phenotype, which could be used as a model animal for disease.

Cells in the individual animal have been so differentiated as to easily effect each function. Each cell can be distinguished not only by its location and morphology, but also immunohistologically with the existence or nonexistence of a marker protein. Alternatively, even if it is difficult to distinguish a certain cell by its morphology, it can be still distinguished only by the existence or nonexistence of expression of a certain gene. It is then considered that such cell effects its own function. Accordingly, it can be a fundamental theme in cell biology to study the function of said cell. Abnormality or defect of a cell having a restricted and specialized function would very likely exercise a serious effect on the whole body of an individual animal.

Patent Documents 1 and 2 disclose a cell specific expression vector, and they describe an apoptosis-related gene as one of DNAs expressed by the vector. Patent Document 3 discloses a specific hybrid promoter for regulating a specific expression in a smooth muscle cell, and it describes a protein inducing apoptosis as one example of the same expression. Patent Document 4 discloses a method of initiating a cell-specific apoptosis with regulation by utilizing a cytoplasmic domain of a human Fas receptor.
Patent Document 1: Japanese Patent Publication 2002-335965
Patent Document 2: Japanese Patent Publication 2003-250579
Patent Document 3: Japanese Patent Publication 2002-525109
Patent Document 4: Japanese Patent Publication Hei 9 (1997)-503645

### DISCLOSURE OF THE INVENTION

### PROBLEMS TO BE SOLVED BY THE INVENTION

It has been observed that in some diseases only a specific-type cell is suffering from damage. Parkinson's disease comprises a small number of congenital abnormality cases and a large number of sporadic cases. Death of nigrostriatal cells has been observed in many of these cases. When a model animal of such disease is produced, it seems that it would be more effective to produce a model animal in which only nigrostriatal cells would be dying than to select a method of deleting a certain protein in the model animal.

However, the prior method of inhibiting a cell function by means of over-expression of RNase will not necessarily induce function inhibitory in all of the cells, and the cells themselves will not be killed, either. In a method of expressing proteinous toxin, there is a possibility that a cell would be lysed after its necrosis so as to have an adverse effect on cells surrounding the cell. Furthermore, in a method of introducing a highly drug-resistance gene, it would be necessary to inject toxin directly into a brain by surgery.

There are some points to be overcome for the success of selectively inducing apoptosis in a specific cell in an individual animal. (1) It is necessary to strictly control the provision of an apoptosis-inducing factor. (2) There exists an intracellular protein that will suppress apoptosis in order not to induce an unnecessary apoptosis, providing a suppressing mechanism in various points in cascades. It is therefore necessary to induce apoptosis, prevailing over such suppression mechanism. (3) It is also necessary not to depend on the expression of a Fas receptor and the like in order to induce apoptosis in a variety of cells. (4) Furthermore, since a promoter activity is weak in many cases of the induction of apoptosis by means of knock-in in the gene-modified mouse, a system that can induce apoptosis only by over-expression would not be suitable.

### MEANS FOR RESOLVING PROBLEMS

The present inventor has studied with respect to a method of inducing apoptosis in a specific cell or tissue and/or at a specific time and killing the cell or tissue in order to overcome the above problems: As a result, the inventor has succeeded in the development of a system in which an apoptosis-inducing factor is expressed in an expression promoter-dependent manner, which is not a system wherein a toxin is introduced externally, and a novel protein that can enhance an apoptosis signal used in the system, leading to the completion of the present invention.
Thus, the present invention relates to the following aspects:
1. An apoptosis-inducing factor comprising a fusion protein of a polypeptide comprising FADD or a constituting domain thereof and a protein with a self-assembling activity.
2. The apoptosis-inducing factor according to the above 1, wherein plurality of DEDs of FADD are linked tandem in the polypeptide.
3. The apoptosis-inducing factor according to the above 2, comprising a fusion protein of a polypeptide comprising two DEDs of FADD linked tandem and a lambda phage E protein.
4. An expression vector comprising DNA encoding the apoptosis-inducing factor according to any one of the above 1-3.
5. The expression vector according to the above 4, wherein the DNA encoding the apoptosis-inducing factor according to any one of the above 1-3 is inserted downstream of an inducible promoter, so that the apoptosis-inducing factor will be expressed under the control of the promoter.
6. The expression vector according to the above 5, wherein the inducible promoter is a drug-inducible promoter.
7. The expression vector according to the above 4, wherein the DNA encoding the apoptosis-inducing factor according to any one of the above 1-3 is so inserted as to be expressed in a specific cell or tissue and/or at a specific time.
8. The expression vector according to the above 7, wherein the DNA encoding the apoptosis-inducing factor according to any one of the above 1-3 is so inserted as to be expressed only in an adult nerve cell.
9. An expression vector wherein the DNA encoding the apoptosis-inducing factor according to any one of the above 1-3 is linked downstream of a promoter of a gene that is expressed only in a specific cell or tissue and/or at a specific time, so that the DNA will be expressed under the control of the promoter.
10.An expression vector wherein the DNA encoding the apoptosis-inducing factor according to any one of the above 1-3 is linked downstream of a promoter of a gene that is expressed only in an adult nerve cell, so that the DNA will be expressed under the control of the promoter.
11. A transgenic animal having the expression vector according to any one of the above 7-10 introduced therein.
12. An expression vector wherein the DNA encoding the apoptosis-inducing factor according to any one of the above 1-3 is inserted between loxP in a reverse direction.
13.An expression vector wherein the DNA encoding the apoptosis-inducing factor according to any one of the above 1-3 is inserted between 1ox66 and lox71 in a reverse direction.
14. A method of inducing apoptosis in a specific cell or tissue and/or at a specific time, comprising using the expression vector according to the above 12 or 13 in CRE/loxP system wherein a CRE protein is expressed under the control of a promoter of a gene that is expressed only in a specific cell or tissue and/or at a specific time.
15. The method according to the above 14, wherein the CRE protein is under the control of a promoter of a gene that is expressed only in an adult nerve cell.
16. The method according to the above 15, wherein the gene that is expressed only in an adult nerve cell is KIAA1793.
17.An animal wherein apoptosis is induced in a specific cell or tissue and/or at a specific time by the method according to any one of the above 14-16.
18. The animal according to the above 17, which is a model animal for disease.
19. The animal according to the above 17 or 18, which is a mouse.
20.A method of producing the animal according to any one of the above 17-19, comprising producing a transgenic animal wherein a CRE protein is expressed under the control of a promoter of a gene that is expressed only in a specific cell or tissue and/or at a specific time and a targeting animal having the DNA encoding the apoptosis-inducing factor according to any one of the above 1-3 between loxP introduced therein, respectively, and mating them with each other.

### ADVANTAGES OF TGE INVENTION

Apoptosis can be effectively induced in a specific cell or tissue and/or at a specific time by expressing an apoptosis-inducing factor according to the present invention. Further, by using the method of producing a model animal for disease according to the present invention, a specific cell such as a nerve cell and an immune system cell can be deleted or killed from individual animals such as a mouse. As this method utilizes apoptosis system that occurs originally in a living body, it can kill only desired cells while eliminating the adverse effects due to toxin, unlike the prior method using the toxin. The present method does not require any external provision of an agent comprising the toxin.

### BRIEF DESCRIPTION OF THE DRAWINGS

Fig.1 is a conceptual diagram showing the mechanism of expressing an apoptosis-inducing factor in a specific cell or tissue and/or at a specific time by means of Cre-loxP system.
Fig.2 shows the results of comparative examples between a transient expression of DED+E protein (lambda phage E protein) in a cell and that of FADD protein alone with respect to capability of inducing apoptosis.
Fig.3 shows photomicrographs sequentially taken in the time course of apoptosis occurring in a stably-expressing strain (gene switch-3T3 cell; Invitrogen Co.) introduced with the same plasmid as that used in the transient expression system and induced by the addition of an agent inducing the expression of 2DED+E protein.

### BEST MODE FOR CARRYING OUT THE INVENTION

FADD (Fas-associated death domain) is a protein that binds to a death domain located in an intracellular region of a Fas receptor, and comprises a death domain (DD) in its C end region and a death effector domain (DED) in its N end region. A precursor of Caspase 8 that has the same domain will bind to DED. As a Fas ligand exist aa a trimer, the precursor of Caspase 8 shall accordingly assemble through the Fas receptor and the FADD protein. It is known that as the precursor of Caspase 8 has a weak protein-processing activity, activation of Caspase 8 will be induced by one another's proteolysis of the assembling precursor of Caspase 8, which will in turn activate a pathway of Caspase 3 downstream and finally induce apoptosis (programmed death).

Accordingly, as a polypeptide constituting the apoptosis-inducing factor according to the present invention, FADD or a constituting domain thereof may be used, DED being especially preferred, to which Caspase 8 will bind. Furthermore, it is preferred that plurality of DEDs, for example two DEDs are linked tandem together. As there is no limitation with respect to an origin (animal variety) of FADD, those derived from mammalian including human and rodent such as a mouse being preferred.

As another member constituting the apoptosis-inducing factor according to the present invention, a protein with a self-assembling activity may be any protein known for those skilled in the art as one having a self-assembling activity in a cell. It includes, for example, a coat protein of phage or virus such as lambda phage E protein and M13 phage g8 protein; human and mouse Mx proteins and their homologous dynamin protein; and cytoskeleton-related factors such as actin protein and tubulin protein.

The "self-assembling activity" in this specification means an activity that will assemble the polypeptide comprising FADD or the constituting domain thereof, which will result in the binding of the precursor of Caspase 8 to FADD or its domain, causing activation of Caspase 8 by one another's proteolysis of the assembling precursor of Caspase 8, activation of a pathway of Caspase 3 downstream, and induction of apoptosis (programmed death).

The above two members constituting the apoptosis-inducing factor according to the present invention, or each constituting domain of FADD may be directly linked with each other, or may alternatively constitute the fusion protein via a suitable linker. Other members such as a peptide may be added to N end and/or C end of the fusion protein as long as the desired advantages are effected.

Furthermore, the polypeptide comprising FADD or the constituting domain thereof and the' protein with the self-assembling activity such as lambda phage E protein according to the present invention may be a polypeptide having an amino acid sequence wherein one or a few amino acids are deleted, substituted or added with respect to their original (wild-type) amino acid sequences, as long as they substantially possess their own (biological) activity. Such polypeptide may be easily prepared by well known methods such as site-specific mutation, genetic homologous recombination, primer extension method and PCR, or any optional combinations thereof.

In order for the above polypeptide or protein to have substantially the same biological activity, it is possible to make a substitution among amino acids belonging to the same group (polar, non-polar, hydrophobic, hydrophilic, positive-charged, negative-charged, or aromatic amino acid group) in the amino acids that constitute the present polypeptide. Alternatively, it is desirable to keep amino acids that are included in a functional domain.

Furthermore, the above two members that constitute the apoptosis-inducing factor according to the present invention may be one encoded by a DNA that hybridizes under stringent conditions with a DNA encoding the polypeptide comprising FADD or the constituting domain thereof and the protein with the self-assembling activity such as lambda phage E protein, more specifically, with a DNA having a complimentary base sequence to said DNA, and shows substantially the function of the above polypeptide.

Hybridization may be performed in accordance with a method described in, for example, Current protocols in molecular biology (edited by Frederick M. Ausubel et al., 1987). If a commercial library is used it may be done according to a method described in instructions attached thereto.

The phrase "stringent conditions" in this specification means conditions under which Southern blot hybridization is carried out in an aqueous solution containing 1mM NaEDTA, 0.5M Na₂HPO₄ (pH 7.2) and 7% SDS at 65°C, followed by the washing of a membrane with an aqueous solution containing 1mM NaEDTA, 40mM Na₂HPO₄ (pH 7.2) and 1% SDS at 65°C. The same stringency may be obtained by using other conditions.

Accordingly, the DNA that hybridizes under stringent conditions with the DNA having the complimentary base sequence to the DNA encoding the polypeptide comprising FADD or the constituting domain thereof and the protein with the self-assembling activity such as lambda phage E protein include a DNA having identity (homology) on an average of about 80% or more, preferably about 90% or more, more preferably about 95% or more to the whole base sequence.

The information about the genes of each member constituting the apoptosis-inducing factor according to the present invention is known for those skilled in the art, and the DNA encoding the same factor may be easily cloned based on said information.

For example, the gene information about human FADD, mouse FADD and lambda phage E protein may be found by referring to NM_003824, U50406, and NC_001416, respectively. Each of them may be searched with GenBank accession number in http://www.ncbi.nlm.nih.gov/entrez/query.fcgi?CMD=search&DB=nucleotide.

The above DNA may be cloned by preparing a synthetic DNA primer with an appropriate nucleotide sequence such as a part of the polypeptide of the present invention, and amplifying it with an appropriate library by means of PCR. The same DNA may be alternatively selected from DNAs integrated into appropriate vectors by means of hybridization with a DNA fragment or synthetic DNA encoding the whole region or part of the present polypeptide.

Hybridization may be performed in accordance with a method described in, for example, Current protocols in molecular biology (edited by Frederick M. Ausubel et al., 1987). If a commercial library is used it may be done according to a method described in instructions attached thereto.

The DNA thus cloned and encoding the polypeptide may be directly used, or optionally digested with a restriction enzyme or tagged with a linker for use. The present DNA may have a translation initiation codon "ATG" at its 5'-end, and a translation termination codon, "TAA", "TGA" or "TAG" at its 3' end. These codons may be also added by using an appropriate synthetic DNA adapter.

The expression vector may be constructed by any known method in the art. For example, it is made by (1) excising a DNA fragment encoding the fusion protein comprising the polypeptide comprising FADD or the constituting domain thereof and the protein with the self-assembling activity, and (2) ligating the DNA fragment downstream of the promoter in a suitable expression vector.

The promoter used in the present expression vector includes such an inducible promoter as its promoter activity will be induced by the addition of a drug (an inducing agent) such as mifepristone, tetracycline, and interferon. Such expression vector is commercially available, for example, as "GeneSwitch system" from Invirogen Co..

The expression vector according to the present invention is characterized by that the DNA encoding the apoptosis-inducing factor according to the present invention is so inserted as to be expressed in a specific cell or tissue such as an adult nerve cell and/or at a specific time.

For example, the expression vector according to the present invention is so constructed that the DNA encoding the apoptosis-inducing factor is linked downstream of a promoter of a gene that is expressed only in a specific cell or tissue and/or at a specific time, so that the DNA will be expressed under the control of the promoter.

The promoter used in the present expression vector includes a cell specific promoter. For example, (1) by using a promoter specifically inducible in a tumor cell, the expression vector according to the present invention may be used instead of a toxin in a missile therapy of cancer so as to provide a safe therapy to induce apoptosis specifically in cancer cells. (2) By using an interferon-inducible promoter, it may be used in the treatment of HIV and the like as it can specifically kill cells infected with viruses. (3) By using a promoter specifically inducible in a immune cell such as Th2, it may be used in the treatment of pollen allergy as it can specifically kill Th2 upon its injection into blood. (4) By using a lipocyte-specific promoter, it may be used in dieting as it can induce apoptosis specifically in the lipocyte.

Another aspect of the expression vector according to the present invention is one that utilizes a known Cre-loxP system. Thus, as is used as a conventional means for a conditional knockout, gene recombination will occur in a specific cell or tissue and/or at a specific time due to a CRE protein. Particularly, the CRE protein is expressed under the control of a promoter of a gene that is expressed under the control of a promoter of a human gene that is expressed only in an adult nerve cell, such as KIAA1793 (GenBank AB058696), neuron-specific enolase (NSE) promoter, neurofilament-H (mNF-H) promoter and the like so as to induce the recombination of a gene at loxP site and express the apoptosis-inducing factor according to the present invention.

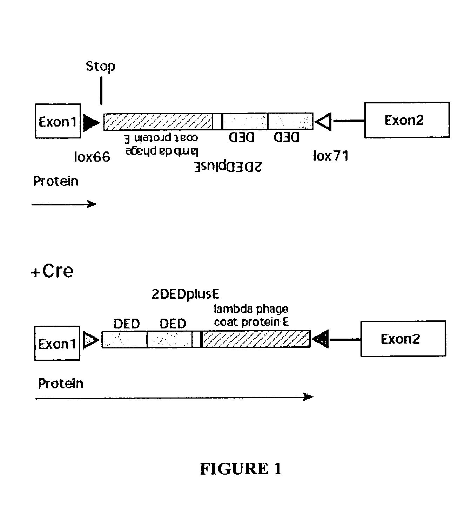

Preferably, the DNA encoding the apoptosis-inducing factor according to the present invention is inserted between loxP in a reverse direction. And when the combination of a mutation type of the loxP site, lox66 and lox71 (Oberdoerffer, P., Otipoby, K. L., Maruyama, M., and Rajewsky, K. 2003, Unidirectional Cre-mediated genetic inversion in mice using the mutant loxP pair lox66/lox71, Nucleic Acids Res, 31, e140., and Schnutgen, F., Doerflinger, N., Calleja, C., Wendling, O., Chambon, P., and Ghyselinck, N. B. 2003, A directional strategy for monitoring Cre-mediated recombination at the cellular level in the mouse, Nat Biotechnol, 21, 562-565.) is used, said DNA that is first located in a reverse direction for an upstream promoter will be revised by gene recombination in response to the provision of the CRE protein into a configuration wherein the apoptosis-inducing factor may be expressed (Fig. 1).

The promoter located upstream of the DNA encoding the apoptosis-inducing factor are preferably any cell or tissue specific promoter known for those skilled in the art, such as L7/pcp-2 promoter, dopamine tansporter promoter, serotonin transporter promoter and Ibal (gamma interfron-inducible) promoter expressed in microglia and macrophage. Such promoter may be the same as those used in the expression of the CRE protein.

Accordingly, apoptosis can be induced in a specific cell or tissue and/or at a specific time using the above expression vector in CRE/loxP system.

The expression vector may be easily prepared by using, for example, a plasmid derived from E. coli such as pBR322, pBR325, pUS18, pUS118; a plasmid derived from Bacillus subtilis such as pUB110, pTP5, pC194; a plasmid derived from yeast such as pSH19 and pSH15; bacteriophage such as lambda phage; animal vruses such as retrovirus, vaccinia virus and baculovirus.

Other elements known in the art such as an enhancer, a splicing signal, a polyadenylation signal, a selection marker and SV40 replication origin may be added to the expression vector, depending on the kind a host cell and the like.

The transfomation of the host cell with the expression vector according to the present invention may be carried out in accordance with a method known in the art such as those described in the following articles:
Proc. Natl. Acad. Sci., USA vol.69, 2110 (1972); Gene, vol.17, 107(1982), Molecular & General Genetics, vol.168, 111 (1979); Methods in Enzymology, vol. 194, 182-187 (1991); Proc. Natl. Acad. Sci., USA vol.75, 1929 (1978); Cell Engneering, additional volume 8, "New Cell Engineering experimental protocols, 263-267 (published by Shu-junn Co.); and Virology vol.52 456 (1973).

A transgenic animal having the expression vector according to the present invention introduced therein may be produced by any method known for those skilled in the art, for example, microinjection of said expression vector into a fertilized egg.

Furthermore, by using the method of inducing apoptosis in a specific cell or tissue and/or at a specific time in CRE/loxP system, an animal wherein apoptosis is induced in a specific cell or tissue and/or at a specific time may be produced. More specifically, such animal may be produced by producing a transgenic animal wherein a CRE protein is expressed under the control of a promoter of a gene that is expressed only in a specific cell or tissue and/or at a specific time and a targeting animal having the DNA encoding the apoptosis-inducing factor according to the present invention between loxP introduced therein, respectively, and mating them with each other in accordance with a conventional way.

There is no limitation with respect to a kind of the animal, which includes mammalians such as rodents comprising a mouse, birds comprising a chicken, and fishes. These animals are useful as a model animal for disease. It is preferable to use the FADD or a constituting domain thereof in the expression vector that is derived from the same animal as that to be transformed with said vector.

The present invention will by further explained by the following examples, which do not limit the scope of the present invention. The genetic procedures in the examples are done in accordance with those described in Current protocols in molecular biology (edited by Frederick M. Ausubel et al., 1987).

### Example 1

### Transient expression cell system

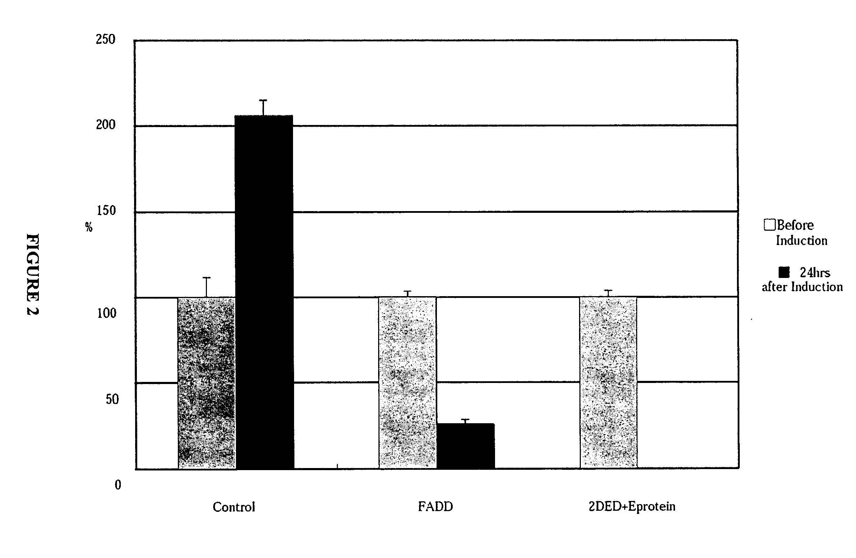

2DED + E protein (lambda phage E protein) and that of FADD protein alone were expressed in a cell and compared with other with respect to their capability of inducing death of cell. As the expression of an apoptosis-inducing protein in a cell would cause apoptosis, GeneSwitch system of Invitrogen Co. was used, in which the expression of FADD protein and the like was suppressed under non-induction, but the expression would occur immediately upon the addition of an inducing agent (Wang Y, O'Malley BW Jr., Tsai SY, O'Malley BW., Proc. Acad. Sci. USA (1994) 91:8180-8184). Furthermore, a fluorescent protein, GFP, was used for comparison in this transient expression system in order to distinguish death of cell due to apoptosis from an un-transformed state. A plasmid comprising both the FADD protein and the like under the control of an inducible promoter (GAL4 UAS) and the GFP protein under the control of an ever-expressing promoter (CMV promoter) was constructed and introduced into NIH3T3 cell using FuGENE6 transfection agent (Roche). After 24-hour culture, the cells were observed under a fluorescence microscope and the number of cells expressing GFP protein was counted. Furthermore, after the addition of an inducing agent (mifepristone; a final concentration of 10 nM), the culture was continued for 24 hours. The were again observed under the fluorescence microscope and the number of cells expressing GFP protein was counted. A ratio of decrease in the number of the cells expressing GFP protein due to the expression of the FADD protein and the like so as to induce apoptosis (cell death) was calculated as taking the initial number of the cells expressing GFP protein as "100%." The results are shown in Fig.2

As shown in Fig.2, the cells expressing GFP protein were not killed in a control experiment, but they were increased at 48 hours after the transfection. While apoptosis occurred in about 75% of the cells at 24 hours after the in the case of the expression of FADD alone, it was observed that apoptosis occurred in almost 100% of the cells in the case of the expression of 2DED+E protein. These results indicated that 2DED+E protein has a stronger apoptosis-inducing capability than FADD alone.

### Example 2

### Integration into genome

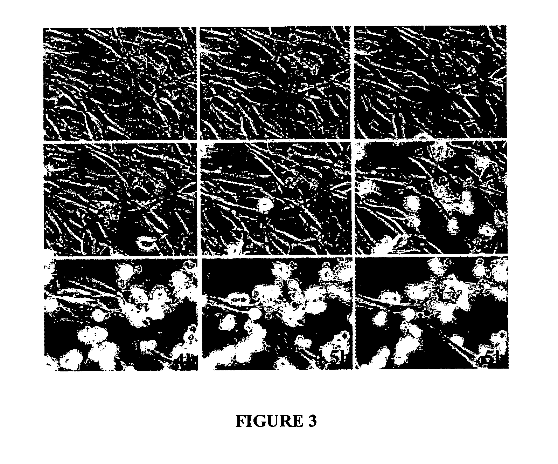

The same plasmid as that used in the transient expression system was mixed with pMClneoPolyA plasmid (Stratagene Co.) at a ratio of 1:20, and introduced into the GeneSwitch-3T3 cell with the above transfection agent. After the addition of antibiotics G418 (Invitrogen CO.) into a culture medium, the cell was cultured for 2 weeks. Emerging antibiotics-resistance strains were isolated so as to obtain a stably-expressing strain wherein the plasmid had been integrated into a chromosome. The agent inducing the expression of 2DED+E protein (mifepristone) was added to the resulting stably-expressing strain at a final concentration of 10 nM. Photomicrographs sequentially taken in the time course of apoptosis occurring in the stably-expressing strain are shown in Fig.3. Each photo shows appearance taken every 30 min after the lapse of one hour from the induction (the upper section from left to right: at 1, 1.5 and 2 hours, each, after the induction, the middle section from left to right: at 2.5, 3, 3.5 hours, each, after the induction, the lower section from left to right: at 4, 4.5 and 5 hours, each, after the induction). It is observed that apoptosis started to occur with morphological change characteristic to apoptosis in each cell at 2.5 hours after the induction. Apoptosis occurred in almost all of the cells at 5 hours after the induction and the cells were removed from a petri dish. The time required for apoptosis to occur in the case of 2DED + E protein was much shorter than that in the case of a typical apoptosis-inducing agent (epoxide, for example). Such rapidness in progress of apoptosis due to 2DED + E protein is a clear evidence that 2DED + E protein itself could enhance the apoptosis-inducing activity.

In order to verify that the change of the cells observed in Examples 1 and 2 was attributed to apoptosis via Caspase 3, an indirect immunofluorescent antibody method was carried out using an antibody specifically detecting an active form of Caspase 3 (Cell Signaling Technology Co.). The method showed that Caspase 3 started to be activated at 2.5 hours after the induction and was fully activated at 3 hours after the induction. Observation of morphological change of nuclei with DAPI stain showed their aggregation and fragmentation, which are characteristic features of apoptosis. Furthermore, cytoplasma also showed morphological change typically seen in apoptosis. These facts clearly showed that 2DED + E protein induced typical apoptosis via Caspase 3.

### INDUSTRIAL APPLICABILITY

The model animal for disease provided by the present invention can be advantageously used in screening system of a drug. The present invention also makes it possible to study the action of a specific cell at an individual level so as to reveal its function. Furthermore, accumulation of knowledge about the function of the specific cell will make it possible to identify a cell type that is useful for varisou industries including medical industry.

The applications of the present invention may be foundas below:
(1) Production of experimental animals such as those lacking granule cells in cerebellum, nerve cells such as Purkinie cell or astroglial cell, or microglial cell; model animals showing Alzheimer's disease-like symptoms due to rough death of nerve cells in cerebral cortex; model animals for disease lacking a specific nerve cell in olfactory brain, or a part of brain such as corpus amygdaloideum, hippocampus, hypothalamus and corpus pineale; model animals for disease lacking immune cells, B cell or T cell (Th1 or Th2); and model animals of muscular dystrophy-like disease due to expression of an exotic virus.
(2) It is a problem in cell differentiation of ES cells and the like in regeneration medicine how to remove other cells than desired ones. In such case, it would be possible remove unnecessary cells by expressing the apoptosis-inducing factor according to the present invention under the control of a promoter of a gene expressed in the unnecessary cells.
(3) In the production of knockout mouse using ES cell, the apoptosis-inducing factor according to the present invention may be expressed under the control of the promoter for induction of tetracycline instead of a negative selection such as a conventional gene of thymidine kinase or diphteria toxin
(4) It would be possible to provide a permanent cure of scabies by killing scabbed epidermic cells.
(5) It could be applied in permanent alopecia due to specific expression in radix pili.

## Claims

1. An apoptosis-inducing factor comprising a fusion protein of a polypeptide comprising FADD or a constituting domain thereof and a protein with a self-assembling activity.

2. The apoptosis-inducing factor according to Claim 1, wherein plurality of DEDs of FADD are linked tandem in the polypeptide.

3. The apoptosis-inducing factor according to Claim 2, comprising a fusion protein of a polypeptide comprising two DEDs of FADD linked tandem and a lambda phage E protein.

4. An expression vector comprising DNA encoding the apoptosis-inducing factor according to any one of Claim 1-3.

5. The expression vector according to Claim 4, wherein the DNA encoding the apoptosis-inducing factor according to any one of Claim 1-3 is inserted downstream of an inducible promoter, so that the apoptosis-inducing factor will be expressed under the control of the promoter.

6. The expression vector according to Claim 5, wherein the inducible promoter is a drug-inducible promoter.

7. The expression vector according to Claim 4, wherein the DNA encoding the apoptosis-inducing factor according to any one of Claim 1-3 is so inserted as to be expressed in a specific cell or tissue and/or at a specific time.

8. The expression vector according to Claim 7, wherein the DNA encoding the apoptosis-inducing factor according to any one of Claim 1-3 is so inserted as to be expressed only in an adult nerve cell.

9. An expression vector wherein the DNA encoding the apoptosis-inducing factor according to any one of Claim 1-3 is linked downstream of a promoter of a gene that is expressed only in a specific cell or tissue and/or at a specific time, so that the DNA will be expressed under the control of the promoter.

10. An expression vector wherein the DNA encoding the apoptosis-inducing factor according to any one of Claim 1-3, is linked downstream of a promoter of a gene that is expressed only in an adult nerve cell, so that the DNA will be expressed under the control of the promoter.

11. A transgenic animal having the expression vector according to any one of Claim 7-10 introduced therein.

12. An expression vector wherein the DNA encoding the apoptosis-inducing factor according to any one of Claim 1-3 is inserted between loxP in a reverse direction.

13. An expression vector wherein the DNA encoding the apoptosis-inducing factor according to any one of Claim 1-3 is inserted between lox66 and lox71 in a reverse direction.

14. A method of inducing apoptosis in a specific cell or tissue and/or at a specific time, comprising using the expression vector according to Claim 12 or 13 in CRE/loxP system wherein a CRE protein is expressed under the control of a promoter of a gene that is expressed only in a specific cell or tissue and/or at a specific time.

15. The method according to Claim 14, wherein the CRE protein is under the control of a promoter of a gene that is expressed only in an adult nerve cell.

16. The method according to Claim 15, wherein the gene that is expressed only in an adult nerve cell is KIAA1793.

17. An animal wherein apoptosis is induced in a specific cell or tissue and/or at a specific time by the method according to any one of Claim 14-16.

18. The animal according to Claim 17, which is a model animal for disease.

19. The animal according to Claim 17 or 18, which is a mouse.

20. A method of producing the animal according to any one of Claim 17-19, comprising producing a transgenic animal wherein a CRE protein is expressed under the control of a promoter of a gene that is expressed only in a specific cell or tissue and/or at a specific time and a targeting animal having the DNA encoding the apoptosis-inducing factor according to any one of Claim 1-3 between loxP introduced therein, respectively, and mating them with each other.
